# EUROPÄISCHE PATENTSCHRIFT

(11) **EP 3 134 384 B1**
(45) Veröffentlichungstag und Bekanntmachung des Hinweises auf die Patenterteilung: **07.11.2018**
(21) Anmeldenummer: 15718846.7
(22) Anmeldetag: 21.04.2015
(51) Int. Cl.: C07C 263/10, C08G 18/76

(54) **VERFAHREN ZUR HERSTELLUNG VON ISOCYANATEN IN DIALKYLCARBONATEN ALS LÖSUNGSMITTEL**
METHOD FOR THE PREPARATION OF ISOCYANATES IN DIALKYL CARBONATES AS SOLVENTS
PROCÉDÉ DESTINÉ À LA FABRICATION D'ISOCYANATES DANS DES CARBONATES DE DIALKYLE EN TANT QUE SOLVANT

(30) Priorität: 23.04.2014 EP 14165533
(43) Veröffentlichungstag der Anmeldung: 01.03.2017
(73) Patentinhaber: BASF SE, 67056 Ludwigshafen am Rhein (DE)
(72) Erfinder: HEILIG, Manfred, 67346 Speyer (DE); SCHULZ, Thomas, 01099 Dresden (DE); MATTKE, Torsten, 67251 Freinsheim (DE); NEVEJANS, Filip, B-9170 St.Gillis-Waas (BE); THIELE, Kai, B-2040 Antwerpen 4 (BE); MAIXNER, Stefan, 68723 Schwetzingen (DE)
(86) Internationale Anmeldenummer: PCT/EP2015/058550
(87) Internationale Veröffentlichungsnummer: WO 2015/162106

(56) Entgegenhaltungen:
- GB-A- 1 146 664

## Beschreibung

Die Erfindung betrifft ein Verfahren zur Herstellung von Isocyanaten durch Umsetzung von primären Aminen mit Phosgen in einem Lösungsmittel.

Es ist bekannt, Isocyanate aus Aminen und Phosgen herzustellen. Die Umsetzung wird je nach Art der Amine entweder in der Gasphase oder in flüssiger Phase sowohl diskontinuierlich als auch kontinuierlich durchgeführt (W. Siefken. Liebigs Ann. 562, 75 (1949)). Die kontinuierliche Herstellung von organischen Isocyanaten durch Reaktion von primären organischen Aminen mit Phosgen ist vielfach beschrieben und wird im großtechnischen Maßstab durchgeführt (siehe Ullmanns Enzyklopädie der Technischen Chemie, Band 7 (Polyurethane), 3. neubearbeitete Auflage, Carl Hanser Verlag, München-Wien, S. 76ff (1993)). Insbesondere die aromatischen Isocyanate TDI (Toluylendiisocyanat) und MDI (Methylendiphenyldiisocyanat), PMDI (Polymethylenpolyphenylenpolyisocyanat) und Mischungen von MDI und PMDI sowie die aliphatischen Isocyanate HDI (Hexamethylendiisocyanat) und Isophorondiisocyanat (IPDI) werden großtechnisch hergestellt.

Heutige großtechnische Synthesen der aromatischen Diisocyanate MDI und TDI und der aliphatischen Diisocyanate HDI und IPDI erfolgen fast ausschließlich in kontinuierlichen Verfahren.

Ein kontinuierliches Verfahren zur Durchführung der Reaktion in mehreren, kontinuierlich durchströmten Gefäßen ist beispielsweise in DE 844 896 beschrieben. In der Regel ist die kontinuierliche Ausführungsform des Verfahrens zweistufig. In der ersten Stufe der Phosgenierung wird das Amin mit Phosgen zum entsprechenden Carbamoylchlorid und Chlorwasserstoff und zu Aminhydrochlorid umgesetzt. Die Reaktion zwischen Amin und Phosgen ist sehr schnell, stark exotherm und läuft schon bei sehr niedrigen Temperaturen ab. Um Nebenprodukt- und Feststoffbildung zu minimieren, müssen daher Amin und Phosgen, beide gegebenenfalls mit organischem Lösungsmittel, schnell vermischt werden, weswegen die erste Phosgenierungsstufe in der Regel in einem Mischorgan, vorzugsweise in einer Düse, erfolgt. Die zweite Stufe der Phosgenierung umfasst sowohl die Zersetzung des Carbamoylchlorids zum gewünschten Isocyanat und zu Chlorwasserstoff als auch die Phosgenierung des Aminhydrochlorids zum Carbamoylchlorid. Die Temperatur der zweiten Phosgenierungsstufe ist in der Regel höher als die der ersten.

Als Lösungsmittel wurden bisher chlorierte aromatische Kohlenwasserstoffe wie Dichlorbenzol, Chlorbenzol, Trichlorbenzol oder Mischungen davon, aromatische oder aliphatische Kohlenwasserstoffe wie Toluol, Xylol, Benzol, Pentan, Hexan, Heptan, Octan, Cyclohexan, Biphenyl, Ketone wie 2-Butanon, Methylisobutylketon, Ester wie Diethylisophthalat, Ethylacetat, Butylacetat, Nitrile wie Acetonitril sowie Sulfolan verwendet. Die dabei erzielten Isocyanat-Ausbeuten sind jedoch noch verbesserungsbedürftig. GB 1,146,664 beschreibt ein Verfahren zur Herstellung von organischen Polyisocyanaten durch Phosphogenierung von Aminen und Polyaminen in Mono-, Di-oder Trichlobenzol.

Aufgabe der Erfindung ist es, ein verbessertes Verfahren zur Herstellung von Isocyanaten durch Umsetzung von primären Aminen mit Phosgen in flüssiger Phase bereitzustellen.

Überraschenderweise wurde gefunden, dass sich Dialkylcarbonate als Lösungsmittel für die Phosgenierung von primären Aminen zu Isocyanaten besonders gut eignen. Versuche haben gezeigt, dass in Dialkylcarbonaten als Lösungsmittel höhere Isocyanat-Ausbeuten als in anderen Lösungsmitteln, beispielsweise in Chloraromaten, erzielt werden. Eine höhere Isocyanat-Ausbeute wird auch in Gemischen aus Dialkylcarbonaten und Chloraromaten erzielt. Dies ist umso überraschender, als der Fachmann erwarten würde, dass primäre Amine mit Dialkylcarbonaten zu Urethanen reagieren, wodurch die Isocyanat-Ausbeute verringert werden müsste.

Alternativ zur Erzielung einer höheren Isocyanat-Ausbeute kann auch - bei gleichbleibender Isocyanat-Ausbeute - bei geringerem Phosgen-Überschuss oder bei geringerer Verdünnung gearbeitet werden

Gegenstand der Erfindung ist somit ein Verfahren zur Herstellung von Isocyanaten durch Umsetzung von primären Aminen mit Phosgen in einem Lösungsmittel, dadurch gekennzeichnet, dass das Lösungsmittel ein Dialkylcarbonat enthält und das primäre Amin ausgewählt ist aus der Gruppe bestehend aus Toluylendiamin (TDA), Diphenylmethandiamin (MDA) und Polymethylenpolyphenylenpolyamin (pMDA).

Geeignete Dialkylcarbonate sind symmetrische oder unsymmetrische Di-C₁ - C₆-alkylcarbonate. Bevorzugte Dialkylcarbonate sind Dimethylcarbonat, Diethylcarbonat, Di-n-propylcarbonat und Diisopropylcarbonat.

Das Dialkylcarbonat kann im Gemisch mit einem weiteren Lösungsmittel vorliegen. Geeignete weitere Lösungsmittel sind chlorierte aromatische Kohlenwasserstoffe wie Monochlorbenzol, Dichlorbenzol, Trichlorbenzol oder Mischungen davon, aromatische oder aliphatische Kohlenwasserstoffe wie Toluol, Xylol, Benzol, Pentan, Hexan, Heptan, Octan, Cyclohexan, Biphenyl, Ketonen wie 2-Butanon, Methylisobutylketon, Ester wie Diethylisophthalat, Ethylacetat, Butylacetat, Nitrile wie Acetonitril, sowie Sulfolan.

Bevorzugte weitere Lösungsmittel sind chlorierte aromatische Kohlenwasserstoffe wie Monochlorbenzol, Dichlorbenzol, Trichlorbenzol oder Mischungen davon. Bevorzugt sind Monochlorbenzol und o-Dichlorbenzol. Die weiteren Lösungsmittel können in Mengen von 10 bis 90 Gew.-%, bevorzugt 10 bis 50 Gew.-%, bezogen auf die Summe an Lösungsmitteln (Dialkylcarbonate plus weitere Lösungsmittel) vorliegen.

Bevorzugte Lösungsmittelgemische enthalten 50 bis 100 Gew.-% Dialkylcarbonat, bevorzugt Diethylcarbonat, und 0 bis 50 Gew.-% chlorierte aromatische Kohlenwasserstoffe, besonders bevorzugt Monochlorbenzol und/oder o-Dichlorbenzol.

Die primäre Amine, die in dem erfindungsgemäßen Verfahren umgesetzt werden, sind Toluylendiamin (TDA), Diphenylmethandiamin (MDA) und Polymethylenpolyphenylenpolyamin (PMDA). Diese werden nach dem erfindungsgemäßen Verfahren zu Toluylendiisocyanat (TDI),

Methylendiphenylendiisocyanat (MDI) bzw. Polymethylenpolyphenylenpolyisocyanat (PMDI) umgesetzt. Weitere primäre Amine, die mit dem erfindungsgemäßen Verfahren umgesetzt werden können, sind Isophorondiamin, Hexamethylendiamin oder 1,5-Naphthalindiamin, die zu Isophorondiisocyanat (IPDI), Hexamethylendiisocyanat (HDI) bzw. 1,5-Naphthalindiisocyanat umgesetzt werden.

In einer bevorzugten Ausführungsform des erfindungsgemäßen Verfahrens erfolgt die Umsetzung in mindestens zwei Stufen, wobei die erste Stufe in einem Mischorgan und die zweite Stufe in mindestens einem Verweilzeitapparat durchgeführt wird. Es kann sich eine dritte Stufe anschließen, die in mindestens einem Stofftrennapparat durchgeführt wird. Beispielsweise wird die Herstellung von Methylendiisocyanat (MDI) bevorzugt zweistufig durchgeführt, kann aber auch dreistufig durchgeführt werden. Die Herstellung von Toluylendiisocyanat wird im Allgemeinen dreistufig durchgeführt. Vorzugsweise ist der Druck in jeder nachfolgenden Stufe geringer ist als in der vorherigen Stufe.

In der ersten Stufe des erfindungsgemäßen Verfahrens erfolgt im wesentlichen die Umsetzung des Amins zu Carbamoylchlorid und Aminhydrochlorid, in der zweiten Stufe im wesentlichen die Umsetzung des in der ersten Stufe gebildeten Aminhydrochlorids zu Carbamoylchlorid und in der dritten Stufe im wesentlichen die Spaltung des Carbamoylchlorids zu Isocyanat und Chlorwasserstoff. In der ersten Stufe werden der vorgemischte, das Amin und Lösungsmittel bzw. Lösungsmittelgemisch enthaltende Feedstrom und der Phosgen enthaltende Feedstrom, der weiterhin Chlorwasserstoff und/oder Lösungsmittel enthalten kann, vermischt, wobei das Vermischen der Feedströme im Allgemeinen sehr schnell erfolgt.

Dabei wird die Reaktion zwischen organischem Amin und Phosgen zwei-, drei- oder mehrstufig, in Dialkylcarbonat als inertem Lösungsmittel mit einem Phosgenüberschuss von im Allgemeinen 1 bis 1000 %, bevorzugt 50 bis 450 % Überschuss über die stöchiometrische Phosgenmenge durchgeführt, wobei über jede der Stufen eine Reduzierung des Drucks erfolgt. Die erste Phosgenierungsstufe umfasst einen statischen Mischer, bevorzugt eine Düse. Der Druck vor der Düse beträgt vorzugsweise 3 bis 70 bar, insbesondere 15 bis 45 bar. Die Druckdifferenz über die Düse ist mindestens 0,5 bar. Die Temperatur in der ersten Stufe beträgt bevorzugt 80 bis 190 °C, insbesondere 90 bis 150 °C. Die zweite Stufe umfasst einen oder mehrere Verweilzeitapparate, bevorzugt einen Verweilzeitapparat, der bei einem Druck von 1,5 bis 35 bar, bevorzugt 2 bis 25 bar betrieben wird. Hinter der Düse wird über eine Armatur oder eine andere dafür geeignete Einrichtung auf den Druck des Verweilzeitapparats der zweiten Stufe entspannt. Es kann allerdings auch der natürliche Druckverlust der Düse zur Druckreduzierung verwendet werden.

Auch kann der Reaktor der ersten Stufe in den Reaktor der zweiten Stufe integriert werden. Insbesondere kann eine Mischdüse in die Gasphase oder bevorzugt in die Flüssigphase des zweiten Reaktors eintauchen, d.h. sich ganz oder teilweise in ihr befinden. Es kann auch der Austrag der Düse mit einer Rohrleitung, einem Tauchrohr oder einem Einsteckrohr in die Gas- oder bevorzugt in die Flüssigphase des Reaktors der zweiten Stufe geführt werden.

Die Temperatur der zweiten Stufe beträgt im Allgemeinen 80 bis 190 °C, bevorzugt 90 bis 150 °C. Als Reaktortyp für die zweite Stufe kommen Rohrreaktoren, Rührkessel, nicht gerührte Verweilzeitapparate, Phasentrennapparate und andere Apparate in Betracht. Der Reaktor kann eine beheizbare Reaktorwand oder einen innenliegenden Wärmetauscher aufweisen. Der Reaktor kann auch mit einem Umpumpkreislauf versehen sein, wobei dieser wiederum einen Wärmetauscher zur Einstellung der Reaktortemperatur enthalten kann. Im Fall eines Rührkessels, eines nicht gerührten Verweilzeitapparats oder gegebenenfalls auch bei einem Phasentrennapparat wird bevorzugt die Flüssigphase standgeregelt und die Gasphase druckgeregelt in den Reaktor der dritten Stufe entspannt. Es kann jedoch auch die Gasphase, hauptsächlich enthaltend Phosgen, Chlorwasserstoff und gegebenenfalls Lösungsmittel, direkt zur Aufarbeitung, wie Auftrennung in Phosgen, Chlorwasserstoff und Lösungsmittel oder in Gemische davon, geführt werden. Der Verweilzeitreaktor der zweiten Stufe kann je nach gewünschter Verweilzeit und Kapazität der Anlage durch größere Abmessungen und Volumina gekennzeichnet sein, was unter Kosten- oder Sicherheitsaspekten, wie Phosgen-Holdup bei hohem Druck, nachteilig sein kann. In diesem Fall kann der Reaktor der zweiten Stufe auch durch zwei oder mehrere gleichartige oder auch unterschiedliche Reaktoren und Reaktortypen, die parallel oder gegebenenfalls zur Beeinflussung des Verweilzeitspektrums auch in Serie geschaltet sein können, realisiert werden.

Der Reaktor der gegebenenfalls vorhandenen dritten Stufe des erfindungsgemäßen Verfahrens wird bei einem Druck von 1,5 bis 20 bar, bevorzugt bei 2 bis 16 bar, betrieben. Hinter dem Verweilzeitreaktor der zweiten Stufe wird über eine Armatur oder eine andere dafür geeignete Einrichtung auf den Druck des dritten Reaktors entspannt. Gegebenenfalls kann auch ein natürlicher Druckabfall genutzt werden.

Die Temperatur der dritten Stufe beträgt im Allgemeinen 80 bis 190 °C. Als Reaktortyp für den dritten Reaktor wird eine Kolonne, insbesondere eine Reaktionskolonne verwendet, wie sie beispielsweise in WO 99/54289 beschrieben ist. Die Sumpftemperatur beträgt im Allgemeinen 80 bis 190 °C und die Kopftemperatur 50 bis 120 °C. Die als Reaktor der dritten Stufe eingesetzte Kolonne kann auch dazu benutzt werden, um das überschüssige Phosgen aus der Reaktionsmischung zu entfernen. Damit der Reaktor der dritten Stufe nicht nachteilig groß ist, kann der Reaktor der dritten Stufe auch durch zwei oder mehrere gleichartige oder auch unterschiedliche Kolonnen, die in Serie geschaltet sind, realisiert werden.

Der Sumpfaustrag der Reaktionskolonne wird mit den üblichen Methoden zur Entfernung gegebenenfalls noch vorhandenen Phosgens und zur Abtrennung des Lösungsmittels aufgearbeitet. Anschließend wird im Falle der Herstellung von TDI das Roh-TDI einer Schwersiederabtrennung und Reindestillation unterworfen. Aus den Brüden der Reaktionskolonne und gegebenenfalls des Verweilzeitreaktors der zweiten Stufe werden in bekannter Weise Phosgen, Chlorwasserstoff und gegebenenfalls Lösungsmittel abgetrennt und gegebenenfalls zurückgeführt.

Die Erfindung wird durch die nachstehenden Beispiele näher erläutert.

### Beispiele

### Beispiel 1 (Vergleichsbeispiel)

In einem thermostatischen Rührbehälter wurden 1300 g Monochlorbenzol und 130 g Phosgen bei 50 °C vorgelegt. Innerhalb 1 h wurde eine Mischung aus 100 g PMDA und 1300 g Monochlorbenzol in die Vorlage über ein Tauchrohr dosiert, wobei die Temperatur auf 50 °C gehalten wurde. Die Gasphase wurde über einen Trockeneiskühler mit Rücklauf in eine Waschkolonne geleitet. Nach beendeter Dosierung wurde die Reaktionsmischung auf ca. 120 °C aufgeheizt und wurden anschließend Teile des Lösungsmittels und Restphosgen abgedampft. Aus der verbliebenen Reaktionsmischung wurden anschließend in einem Rotationsverdampfer unter Vakuum (50 mbar) bei zunächst 100 °C und abschließend bei 180 °C für jeweils eine Stunde Lösungsmittelreste abgetrennt. Anschließend wurde die NCO-Zahl der Probe (Gewichtsanteil NCO-Gruppen in der Probe) mittels Titration als Maß für die Ausbeute zu 31,8 Gew.-% bestimmt.

### Beispiel 2 (erfindungsgemäß)

Der oben beschriebene Versuch wurde wiederholt, wobei in der Vorlage statt 1300 g Monochlorbenzol nun eine Mischung aus 520 g Diethylcarbonat und 780 g Monochlorbenzol vorgelegt wurde. Die ermittelte NCO-Zahl betrug 32,2 Gew.-%.

## Patentansprüche

1. Verfahren zur Herstellung von Isocyanaten durch Umsetzung von primären Aminen mit Phosgen in einem Lösungsmittel, **dadurch gekennzeichnet, dass** das Lösungsmittel ein Dialkylcarbonat enthält und das primäre Amin ausgewählt ist aus der Gruppe bestehend aus Toluylendiamin (TDA), Diphenylmethandiamin (MDA) und Polymethylenpolyphenylenpolyamin (pMDA).

2. Verfahren nach Anspruch 1, **dadurch gekennzeichnet, dass** das Dialkylcarbonat ausgewählt ist aus der Gruppe bestehend aus Dimethylcarbonat, Diethylcarbonat, Di-n-propylcarbonat und Diisopropylcarbonat.

3. Verfahren nach Anspruch 1 oder 2, **dadurch gekennzeichnet, dass** das Dialkylcarbonat im Gemisch mit einem weiteren Lösungsmittel vorliegt.

4. Verfahren nach Anspruch 3, **dadurch gekennzeichnet, dass** das weitere Lösungsmittel ein aromatischer Chlorkohlenwasserstoff ist.

5. Verfahren nach Anspruch 4, **dadurch gekennzeichnet, dass** der aromatische Chlorkohlenwasserstoff ausgewählt ist aus der Gruppe bestehend aus Monochlorbenzol und o-Dichlorbenzol.

6. Verfahren nach Anspruch 4 oder 5, **dadurch gekennzeichnet, dass** das Lösungsmittel 50 bis 100 Gew.-% Diethylcarbonat und 0 bis 50 Gew.-% des aromatischen Chlorkohlenwasserstoffs als weiteres Lösungsmittel, bezogen auf die Summe aus Dialkylcarbonat und weiterem Lösungsmittel, enthält.

7. Verfahren nach einem der Ansprüche 1 bis 6, **dadurch gekennzeichnet, dass** die Umsetzung in mindestens zwei Stufen erfolgt, wobei die erste Stufe in einem Mischorgan und die zweite Stufe in mindestens einem Verweilzeitapparat durchgeführt wird.

8. Verfahren nach Anspruch 7, **dadurch gekennzeichnet, dass** die Umsetzung in mindestens drei Stufen durchgeführt wird, wobei die dritte Stufe in mindestens einem Stofftrennapparat durchgeführt wird, und der Druck in jeder nachfolgenden Stufe geringer ist als in der vorhergehenden Stufe.

## Claims

1. A process for preparing isocyanates by reacting primary amines with phosgene in a solvent, wherein the solvent comprises a dialkyl carbonate and the primary amine is selected from the group consisting of toluenediamine (TDA), diphenylmethanediamine (MDA) and polymethylenepolyphenylenepolyamine (pMDA).

2. The process according to claim 1, wherein the dialkyl carbonate is selected from the group consisting of dimethyl carbonate, diethyl carbonate, di-n-propyl carbonate and diisopropyl carbonate.

3. The process according to claim 1 or 2, wherein the dialkyl carbonate is present in admixture with a further solvent.

4. The process according to claim 3, wherein the further solvent is an aromatic chlorinated hydrocarbon.

5. The process according to claim 4, wherein the aromatic chlorinated hydrocarbon is selected from the group consisting of monochlorobenzene and o-dichlorobenzene.

6. The process according to claim 4 or 5, wherein the solvent comprises from 50 to 100% by weight of diethyl carbonate and from 0 to 50% by weight of the aromatic chlorinated hydrocarbon as further solvent, based on the sum of dialkyl carbonate and the further solvent.

7. The process according to any of claims 1 to 6, wherein the reaction is carried out in at least two stages, with the first stage being carried out in a mixing device and the second stage being carried out in at least one residence apparatus.

8. The process according to claim 7, wherein the reaction is carried out in at least three stages, with the third stage being carried out in at least one materials separation apparatus and the pressure in each successive stage being lower than in the preceding stage.

## Revendications

1. Procédé de fabrication d'isocyanates par mise en réaction d'amines primaires avec du phosgène dans un solvant, **caractérisé en ce que** le solvant contient un carbonate de dialkyle et l'amine primaire est choisie dans le groupe constitué par la toluylène-diamine (TDA), la diphénylméthane-diamine (MDA) et la polyméthylène-polyphénylène-polyamine (pMDA).

2. Procédé selon la revendication 1, **caractérisé en ce que** le carbonate de dialkyle est choisi dans le groupe constitué par le carbonate de diméthyle, le carbonate de diéthyle, le carbonate de di-n-propyle et le carbonate de diisopropyle.

3. Procédé selon la revendication 1 ou 2, **caractérisé en ce que** le carbonate de dialkyle se présente en mélange avec un autre solvant.

4. Procédé selon la revendication 3, **caractérisé en ce que** l'autre solvant est un hydrocarbure chloré aromatique.

5. Procédé selon la revendication 4, **caractérisé en ce que** l'hydrocarbure chloré aromatique est choisi dans le groupe constitué par le monochlorobenzène et l'o-dichlorobenzène.

6. Procédé selon la revendication 4 ou 5, **caractérisé en ce que** le solvant contient 50 à 100 % en poids de carbonate de diéthyle et 0 à 50 % en poids de l'hydrocarbure chloré aromatique en tant qu'autre solvant, par rapport à la somme du carbonate de dialkyle et de l'autre solvant.

7. Procédé selon l'une quelconque des revendications 1 à 6, **caractérisé en ce que** la réaction a lieu en au moins deux étapes, la première étape étant réalisée dans un appareil de mélange et la deuxième étape étant réalisée dans au moins un appareil à temps de séjour.

8. Procédé selon la revendication 7, **caractérisé en ce que** la réaction est réalisée en au moins trois étapes, la troisième étape étant réalisée dans au moins un appareil de séparation de matière, et la pression dans chaque étape ultérieure étant inférieure à celle dans l'étape précédente.
